# EUROPEAN PATENT APPLICATION

(11) **EP 4 557 303 A1**
(43) Date of publication of application: **21.05.2025**
(21) Application number: 24212051.7
(22) Date of filing: 11.11.2024
(51) Int. Cl.: G16H 20/17, G16H 40/63, G16H 40/67, G16H 50/30, G16H 50/70, A61M 5/14

(54) **DETERMINATION OF CARBOHYDRATE-TO-INSULIN RATIOS**

(30) Priority: 16.11.2023 US 202363599815 P; 16.10.2024 US 202418917224
(71) Applicant: MEDTRONIC MINIMED, INC., Northridge, CA 91325-1219 (US)
(72) Inventor: Roy, Anirban, Northridge, 91325 (US); Grosman, Benyamin, Northridge, 91325 (US); Lintereur, Louis J., Northridge, 91325 (US)
(74) Representative: Zimmermann & Partner Patentanwälte mbB

(57) **Abstract**

Disclosed herein are techniques related to determining medical parameters. In some embodiments, the techniques involve: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.

## Description

### CROSS-REFERENCE TO RELATED APPLICATIONS

This application claims priority to U.S. Provisional Application No. 63/599,815, filed November 16, 2023, and titled "DETERMINATION OF CARBOHYDRATE-TO-INSULIN RATIOS," the entire contents of which is herein incorporated by reference for all purposes.

### TECHNICAL FIELD

The present disclosure relates generally to determination of carbohydrate-to-insulin ratios.

### BACKGROUND

A carbohydrate-to-insulin ratio is a crucial parameter for medication (e.g., insulin) delivery. The carbohydrate-to-insulin ratio may vary substantially from person to person, and it is critical for both safety and medication effectiveness to accurately set the carbohydrate-to-insulin ratio parameter, e.g., for an insulin pump. However, it can be difficult to determine an accurate and safe value, or an accurate and safe range of values.

### SUMMARY

Disclosed herein are techniques related to determination of carbohydrate-to-insulin ratios. The techniques may be practiced in a variety of ways, such as using a processor-implemented method; a system comprising one or more processors and one or more processor-readable media; and/or one or more processor-readable media (e.g., non-transitory processor-readable media).

In one aspect, a processor-implemented method is disclosed. In some embodiments, the processor-implemented method includes: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.

In another aspect, a system is disclosed. The system may include one or more processors and one or more processor-readable media storing instructions which, when executed by the one or more processors, cause performance of one or more operations including: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.

In another aspect, one or more processor-readable media storing instructions are disclosed. In some embodiments, when executed by one or more processors, the instructions cause performance of: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.

In another aspect, a processor-implemented method is disclosed. In some embodiments, the processor-implemented method includes: obtaining a total daily dose (TDD) of insulin for a patient; and determining at least one carbohydrate-to-insulin ratio for the patient, wherein the carbohydrate-to-insulin ratio is less than 25 grams/unit for a TDD less than 10 units, and wherein the carbohydrate to insulin ratio is greater than 5 grams/unit for a TDD for a TDD less than 105 units.

Further disclosed herein are techniques related to determining medical parameters. In some embodiments, the techniques involve: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients

### BRIEF DESCRIPTION OF THE DRAWINGS

The above and other aspects and features of the disclosure will become more apparent in view of the following detailed description when taken in conjunction with the accompanying drawings wherein like reference numerals identify like elements.
FIG. 1 is a diagram of an example of a therapy delivery system, in accordance with aspects of the present disclosure.
FIGS. 2A,2B, 2C, and 2D illustrate graphs of an example function that relates a carbohydrate-to-insulin ratio to a total daily dose (TDD) of insulin that may be used in accordance with some embodiments.
FIG. 3 is a flowchart of an example process for determining at least one carbohydrate-to-insulin ratio for a patient based on a model in accordance with some embodiments.
FIG. 4 is a flowchart of an example process for determining at least one carbohydrate-to-insulin ratio for a patient based on a TDD for the patient in accordance with some embodiments.
FIG. 5 is a diagram of an example of an insulin delivery device, in accordance with aspects of the present disclosure.
FIG. 6 is a block diagram of an example of a computer system, which can be utilized in embodiments as described herein.

### DETAILED DESCRIPTION

Therapeutic substances (e.g., insulin) may be delivered to a diabetic patient to manage, for example, type I or type II diabetes. Delivering appropriate amounts of a therapeutic substance at appropriate times can help maintain glucose levels within a target range (e.g., a euglycemic range) in the body to prevent hyperglycemia or hypoglycemia conditions.

Disclosed herein are techniques for determining at least one carbohydrate-to-insulin ratio (CIR) for a patient. The CIR may be used to determine a dosage of insulin to be delivered to counteract a given number of carbohydrates consumed. For example, given a CIR of*X* grams/unit and a number of consumed carbohydrates of Y grams, an insulin dosage may be determined as *Y*/*X* units. The determined insulin dosage may then be delivered, e.g., by actuating an infusion pump to cause the insulin to be provided to the patient.

In some embodiments, the at least one CIR may be determined based on a model, where parameters of the model were determined based on data associated with a population of patients. For example, the data associated with the population of patients may include one or more CIRs for a given patient and one or more insulin dosage metrics for the patient, where the model relates the one or more CIRs to the one or more insulin dosage metrics. The insulin dosage metrics may include a total daily dose (TDD) of insulin, total bolus dosage of insulin per day, total insulin over a predetermined time range or time period (e.g., mornings, evenings, etc.), or the like.

By using a population of patients to generate the model (e.g., to determine parameters that define the model), the resulting CIRs may be more accurate than CIRs that are conventionally determined. Conventional techniques, such as the 500 Rule, 350 Rule, or the 300 Rule estimate CIR based on a patient's TDD, and these conventional techniques may lead to CIR estimations which are either too conservative or too aggressive, which may cause health and safety problems. For example, the 500 Rule, the 350 Rule, and/or the 300 Rule may over-estimate CIR (e.g., by determining a CIR that is too conservative and/or not aggressive enough) for patients with low TDDs. This may particularly affect pediatric patients, as TDD tends to be correlated with age. Conversely, the 500 Rule,the 350 Rule, and/or the 300 Rule may under-estimate CIR (e.g., determining a CIR that is too aggressive) for patients with high TDDs. This may tend to cause hypoglycemia by causing insulin dosages to be calculated that are too high for a given amount of carbohydrates. However, by using the techniques disclosed herein, CIRs may be determined that are more accurate, e.g., neither too conservative nor too aggressive. It should be understood that although conventional techniques are generally described herein as the 500 Rule, the 350 Rule, and/or the 300 Rule, the techniques disclosed herein differ from these conventional techniques by utilizing a model to predict CIRs based on TDDs based on population-level data, rather than using fixed ratios of CIR to TDD.

In some embodiments, a model that relates CIR to insulin dosage metrics may be used to determine a median CIR for a given insulin dosage. For example, the model may be used to determine, for a patient with a given TDD, the CIR of the median patient with the TDD. Additionally or alternatively, in some embodiments, the model may provide indications of CIRs at any suitable other percentiles. For example, in an instance in which the insulin dosage metric is TDD, the model may be used to determine the 10^{th} percentile CIR that a patient with the TDD uses, the 90^{th} percentile CIR that a patient with the TDD uses, and/or any other percentiles. As used herein, an *N^{th}* percentile CIR refers to the CIR for which *N* percent of patients use the CIR or a lower CIR (or, alternatively, for which 100-N percent of patients use a higher CIR). Using the model to determine various percentiles for a given insulin dosage metric may allow a patient and/or a healthcare provider to determine where a patient's CIR falls with respect to a population of patients having the same or similar insulin dosage metrics. Moreover, the various percentiles may be used to provide an alert or a warning in an instance in which a patient or a healthcare provider inputs a CIR (e.g., to be used as a setting for an infusion pump) that is above or below a given percentile for a corresponding insulin dosage metric. By way of example, in an instance in which the insulin dosage metric used is TDD, and a patient or healthcare provider provides an indication of a CIR that is determined to be outside a predetermined percentile range (e.g., outside of the range of CIRs that fall within the 10^{th}-90^{th} percentiles, outside of the range of CIRs that fall within the 25^{th}-75^{th} percentiles, or the like), the techniques disclosed herein may cause a warning or an alert to be provided. This may allow erroneous entries of CIRs to be detected prior to usage, which may thereby prevent health and safety problems.

The present disclosure is described primarily with respect to insulin delivery systems. Aspects and embodiments of the present disclosure can be practiced with one or more types of insulin (e.g., fast-acting insulin, intermediate-acting insulin, and/or slow-acting insulin). For example, fast-acting insulin may be used for both basal dosages and bolus dosages.

Although the present disclosure is described primarily with respect to insulin delivery systems, the scope of the present disclosure is not limited to insulin delivery systems. Rather, the present disclosure applies to and can be implemented for other therapy systems as well. For example, some techniques of the present disclosure may be adapted for practice in relation to glucagon delivery systems.

Discussions utilizing terms such as, for example, "processing," "computing," "calculating," "determining," "establishing," "analyzing," "checking," or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other non-transitory information storage media that may store instructions to perform operations and/or processes by, e.g., one or more processor or processor apparatus (e.g., system on a chip) or a device associated with such processor(s).

In the context of this disclosure, a "module" may refer to a set of computer-executable instructions and/or a hardware processor configured to execute a set of computer-executable instructions. A hardware processor may be an integrated circuit device associated with a computing device, such as a server or a user device (e.g., a desktop computer, a laptop computer, a tablet computer, a mobile phone, or the like), which is programmable to perform specific tasks. In some embodiments, multiple modules may be implemented as a single module. In some embodiments, a single module may be implemented as multiple modules. In some embodiments, two or more modules may be executable by the same device (e.g., the same computing device or delivery device).

Unless explicitly stated, the methods described herein are not constrained to a particular order or sequence. Additionally, some of the described methods or elements thereof can occur or be performed simultaneously or concurrently.

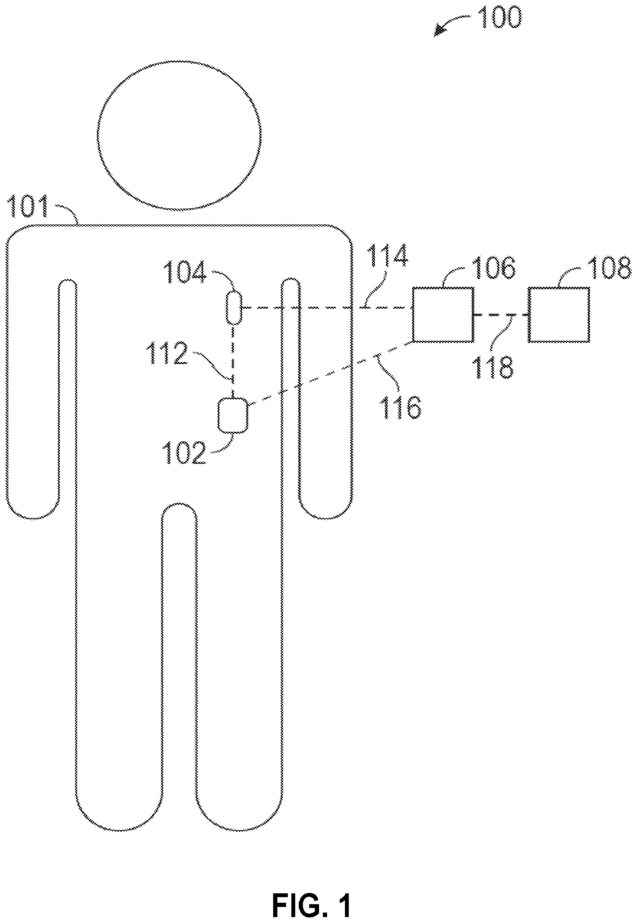

FIG. 1 depicts an example therapy delivery system 100 for a person 101. Components of therapy delivery system 100 may be used to implement one or more blocks of process 300 and/or process 400. System 100 may be an insulin delivery system. The depicted therapy delivery system 100 includes a delivery device 102, a monitoring device 104, a computing device 106, and an optional remote or cloud computing system 108. The delivery device 102, the monitoring device 104, and the computing device 106 may be embodied in various ways, including being disposed in one or more device housings. For example, in some embodiments, all of the devices 102-106 may be disposed in a single device housing. In some embodiments, each of the devices 102-106 may be disposed in a separate device housing. In some embodiments, two or more of the devices 102-106 may be disposed in the same device housing, and/or a single device 102, 104, or 106 may have two or more parts that are disposed in two or more housings. Such embodiments, and combinations thereof, are contemplated to be within the scope of the present disclosure.

FIG. 1 also depicts communications links 112-118. The communications links 112-118 may each be a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a USB connection, and/or another type of physical connection. Wireless connections may include, without limitation, a cellular connection, a Wi-Fi connection, a Bluetooth^{®} connection, a mesh network connection, and/or another type of connection using a wireless communication protocol. Some embodiments of the communication links 112-118 may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 112-118.

Aspects of the insulin delivery system 100 are described below. Further aspects and details may be described in United States Patent Nos.: 4,562,751; 4,685,903; 5,080,653; 5,505,709; 5,097,122; 6,485,465; 6,554,798; 6,558,320; 6,558,351; 6,641,533; 6,659,980; 6,752,787; 6,817,990; 6,932,584; and 7,621,893. The entire contents of each of the foregoing United States Patents are hereby incorporated by reference herein.

The delivery device 102 is configured to deliver a therapeutic substance (e.g., insulin) to a person 101. The delivery device 102 may be secured to the person 101 (e.g., to the body or clothing of the person 101) or may be implanted on or in the body of the person 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 101. The cannula and/or a needle may facilitate delivery of the therapeutic substance to a tissue layer, vein, or body cavity of the person 101. During operation, the actuator, in response to a signal (e.g., a command signal), may drive the delivery mechanism, thereby causing the therapeutic substance to move from the reservoir, through the cannula, and into the body of the person 101.

The components of the delivery device 102 described above are merely provided as examples. The delivery device 102 may include other components, such as, without limitation, a power supply, a communication transceiver, computing resources, and/or user interfaces, among other things. Persons skilled in the art will recognize various implementations of the delivery device 102 and the components of such implementations. All such implementations and components are contemplated to be within the scope of the present disclosure.

With continuing reference to FIG. 1, the monitoring device 104 is configured to detect a physiological condition (e.g., a glucose concentration level) of the person 101 and may also be configured to detect other things. The monitoring device 104 may be secured to the body of the person 101 (e.g., to the skin of person 101 via an adhesive) and/or may be at least partially implanted into the body of the person 101. Depending on the particular location or configuration, the monitoring device 104 may be in contact with biological matter (e.g., interstitial fluid and/or blood) of the person 101.

The monitoring device 104 includes one or more sensors (not shown), such as, without limitation, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker. In the case of electrical sensors, and as persons skilled in the art will understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 101. In some embodiments, the electrical biosignal may include portions of a potential change produced by the heart of the person 101 over time, e.g., recorded as an electrocardiogram, that are indicative of a glucose level of the person 101. In the case of optical sensors, as persons skilled in the art will understand, an optical sensor may be configured to respond to the interaction or binding of a biological marker to a substrate by generating an electrical signal based on change in luminance of the substrate. For example, the substrate may include a material selected to fluoresce in response to contact with a selected biomarker, such as glucose. The fluorescence may be proportional to a concentration of the selected biomarker.

In some embodiments, the monitoring device 104 may include other types of sensors that may be worn, carried, or coupled to the person 101 to measure activity of the person 101 that may influence the glucose levels or glycemic response of the person 101. As an example, the sensors may include an acceleration sensor configured to detect an acceleration of the person 101 or a portion of the person 101, such as the person's hands or feet. The acceleration (or lack thereof) may be indicative of exercise, sleep, or food/beverage consumption activity of the person 101, which may influence the glycemic response of the person 101. In some embodiments, the sensors may include heart rate and/or body temperature, which may indicate an amount of physical exertion experienced by the person 101. In some embodiments, the sensors may include a GPS receiver which detects GPS signals to determine a location of the person 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor shall be referred to as a "sensor signal."

The monitoring device 104 may include components and/or circuitry configured to pre-process sensor signals. Pre-processing may include, without limitation, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, ASICS, integrated circuits, hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 104 may not perform pre-processing.

As used herein, the term "sensed data" shall mean and include the information represented by a sensor signal or by a pre-processed sensor signal. In some embodiments, sensed data may include glucose levels in a person 101, acceleration of a part of the person 101, heart rate of the person 101, temperature of the person 101, and/or geolocation (e.g., GPS location) of the person 101, among other things. The monitoring device 104 may communicate sensed data to the delivery device 102 via communication link 112 and/or to the computing device 106 via communication link 114. Use of sensed data by the delivery device 102 and/or by the computing device 106 will be described later herein.

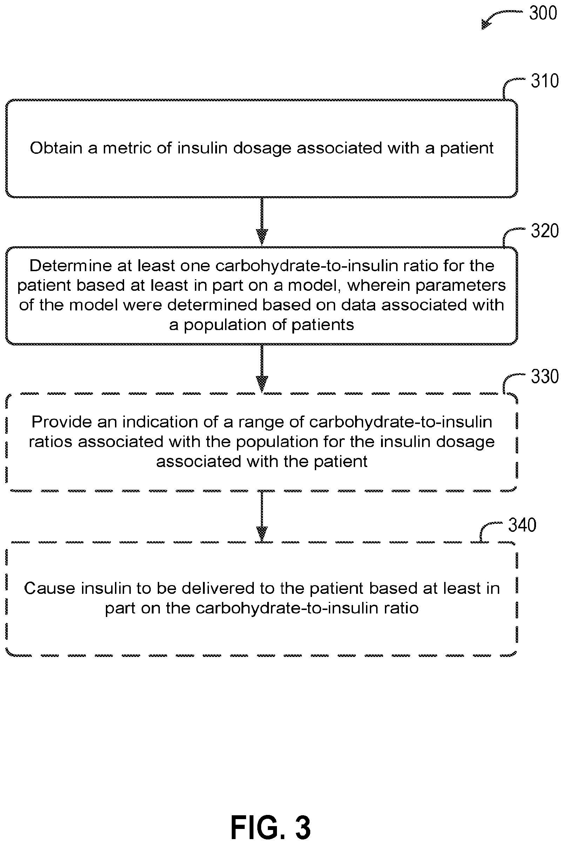

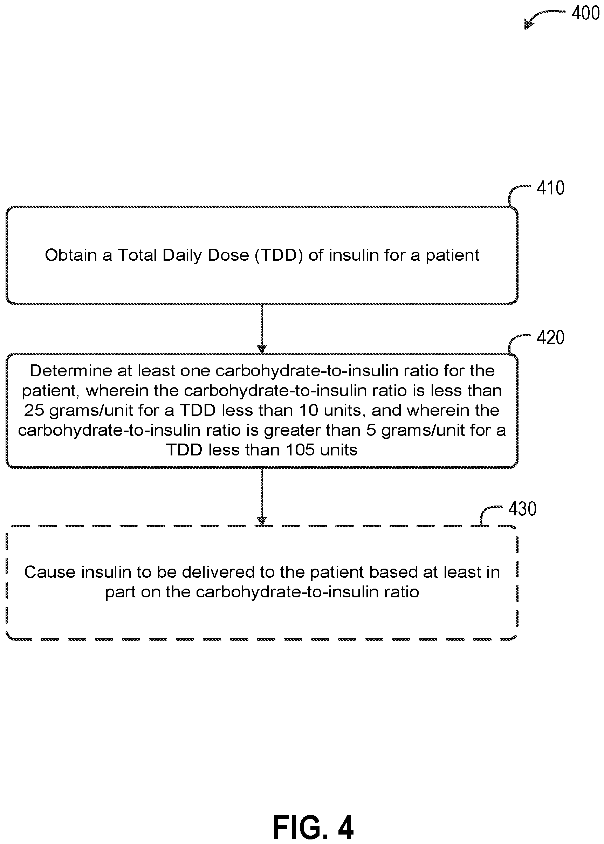

The computing device 106 provides processing capabilities and may be implemented in various ways. In some embodiments, the computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 102. In some embodiments, the computing device 106 may be "processing circuitry" (defined below) that is integrated with another device, such as the delivery device 102. In some embodiments, the computing device 106 may be secured to the person 101 (e.g., to the body or clothing of person 101), may be at least partially implanted into the body of person 101, and/or may be held by the person 101. In some embodiments, computing device 106 may be configured to execute one or more blocks of process 300 and/or process 400, shown in FIGS. 3 and 4 below. For example, in some embodiments, computing device 106 may determine a CIR for a patient based on insulin dosage metrics.

For each of the embodiments of the computing device 106, the computing device 106 may include various types of logic circuitry, including, but not limited to, microprocessors, microcontrollers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the delivery device 102, the monitoring device 104, and the computing device 106 have been described above. One or more of the devices 102-106 may include a user interface (not shown) that presents information to the person 101 and/or receives information from the person 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 101 of sensed data (e.g., glucose level) and/or insulin delivery data (e.g., rates of historic, current, or future insulin delivery) and may present alerts to the person 101. In some embodiments, a user interface may receive inputs from the person 101, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

The following describes communications between the devices 102-106 and cooperation between the devices 102-106 with respect to insulin delivery. As depicted in FIG. 1, and as mentioned above, the devices 102-106 may communicate with each other via communication links 112-116. In some embodiments, the computing device 106 may control operation of the delivery device 102 and/or the monitoring device 104. For example, the computing device 106 may generate one or more signals (e.g., a command signal) that cause the delivery device 102 to deliver insulin to the person 101, e.g., as a basal dosage and/or a bolus dosage. In some embodiments, the computing device 106 may receive data associated with insulin delivery (e.g., insulin delivery data) from the delivery device 102 and/or receive sensed data (e.g., glucose levels) from the monitoring device 104 and may perform computations based on the insulin delivery data, the sensed data, and/or other data to control the delivery device 102. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 106 may communicate dosage commands to the delivery device 102 based on a difference between a current glucose level in the body of the person 101 (e.g., received from the monitoring device 104) and a target glucose level (e.g., determined by the computing device 106). The dosage commands may indicate an amount of insulin to be delivered and/or a rate of insulin delivery and may regulate the current glucose level toward the target glucose level. Examples of closed-loop operations for insulin infusion systems are described in United States Patent Nos.: 6,088,608, 6,119,028, 6,589,229, 6,740,072, 6,827,702, 7,323,142, and 7,402,153, and in United States Patent Application Publication Nos.: 2014/0066887 and 2014/0066889. The entire contents of each of the foregoing patents and publications are hereby incorporated by reference herein.

With continuing reference to FIG. 1, the remote or cloud computing system 108 may be a proprietary remote/cloud computing system or a commercial cloud computing system including one or more server computing devices. The remote/cloud computing system 108 may provide additional computing resources on-demand as needed when the computing resources of a client computing device (e.g., the computing device 106) are not sufficient. The computing device 106 and the remote/cloud computing system 108 may communicate with each other through a communication link 118, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 108 and how to interface with such systems through various types of networks. For example, the remote/cloud computing system 108 may include an array of processing circuitry (defined above) and may execute machine-readable instructions. Such implementations, interfaces, and networks are contemplated to be within the scope of the present disclosure. In some embodiments, remote or cloud computing system 108 may be configured to execute one or more blocks of process 300 and/or process 400, as shown in FIGS. 3 and 4, respectively. For example, in some embodiments, remote or cloud computing system 108 may be configured to determine a CIR for a patient. In some embodiments, remote or cloud computing system 108 may store and/or analyze data from a population of patients used to determine model parameters for a model that relates insulin dosage metrics to a CIR. In some embodiments, remote or cloud computing system 108 may determine the model parameters for the model. In some embodiments, remote or cloud computing system 108 may store a look-up table that includes results from the model, e.g., that indicates CIRs for different values of an insulin dosage metric. In some such embodiments, remote or cloud computing system 108 may transmit any suitable information, such as data from a look-up table, model parameters, and/or a determined CIR to computing system associated with a particular medical device (e.g., computing system 106) for use with the medical device.

An example therapy delivery system has been described above. For convenience, the description below may primarily refer to an insulin delivery system as an example of the therapy delivery system. However, it is intended that any aspect, embodiment, or description relating to an insulin delivery system shall be applicable to a therapy delivery system which delivers a therapy other than insulin.

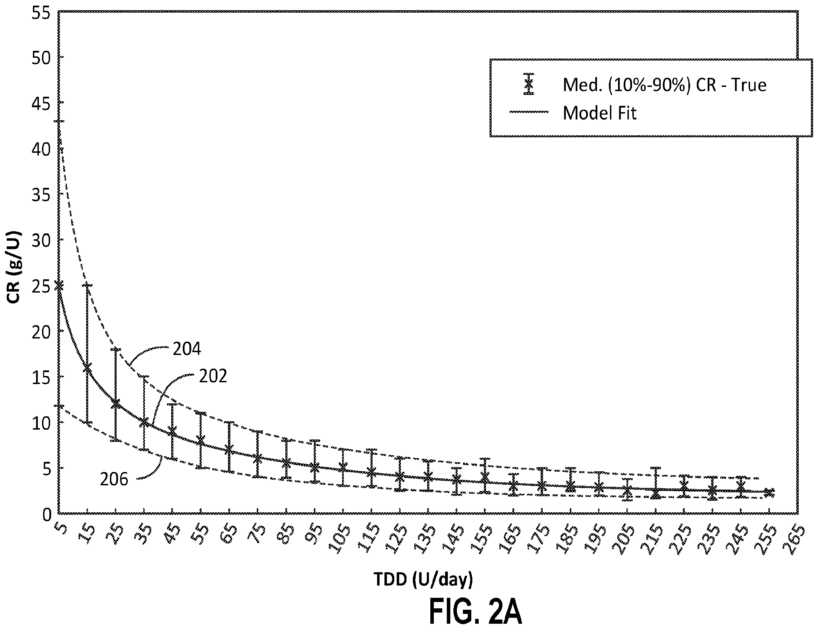

FIG. 2A depicts an example graph that illustrates CIRs as a function of TDD (in units per day). As illustrated, a curve 202 is a model fit to population data, where the model fit was determined based on CIRs of a set of patients based on each patient's TDD. Curve 204 depicts the 90^{th} percentile of CIRs for each TDD, and curve 206 depicts the 10^{th} percentile of CIRs for each TDD. Note that curves 202, 204, and 206 each utilize one or more mathematical functions to approximate an exponential decay. The mathematical functions may include any combination of exponential functions, polynomial functions, or the like. In some embodiments, curve 202 may be generated by fitting the one or more mathematical functions to the median data for each TDD, curve 204 may be generated by fitting one or more mathematical functions to the 90^{th} percentile data for each TDD, and curve 206 may be generated by fitting one or more mathematical functions to the 10^{th} percentile data for each TDD.

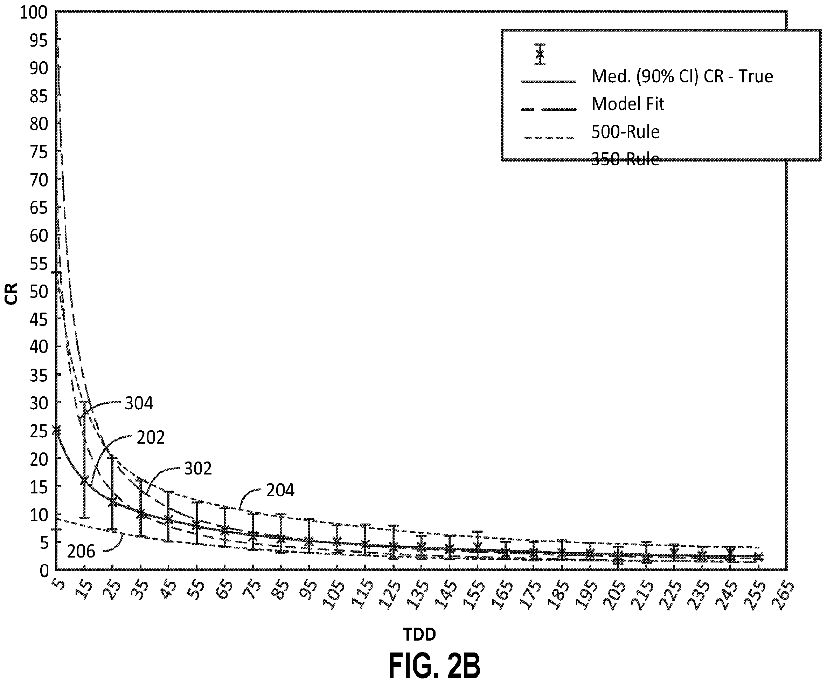

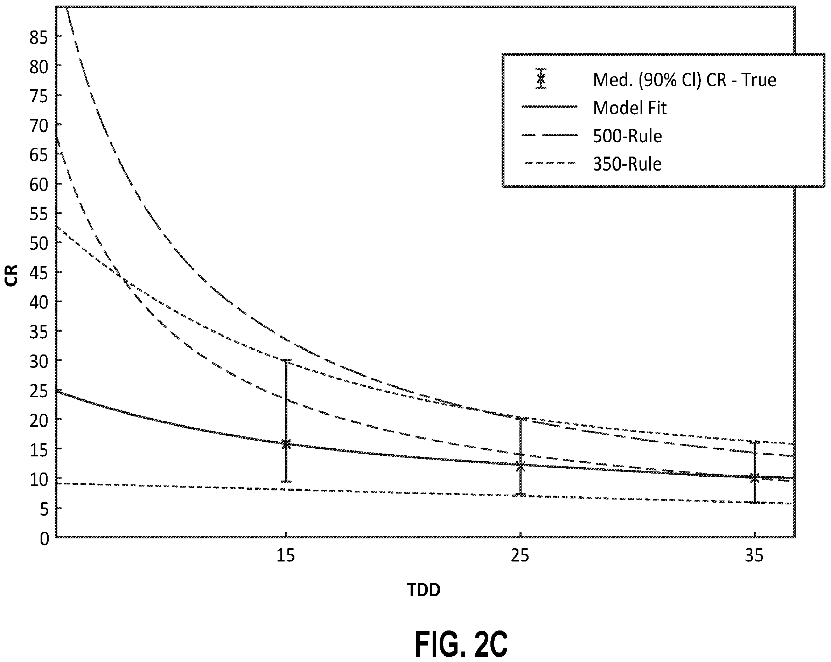

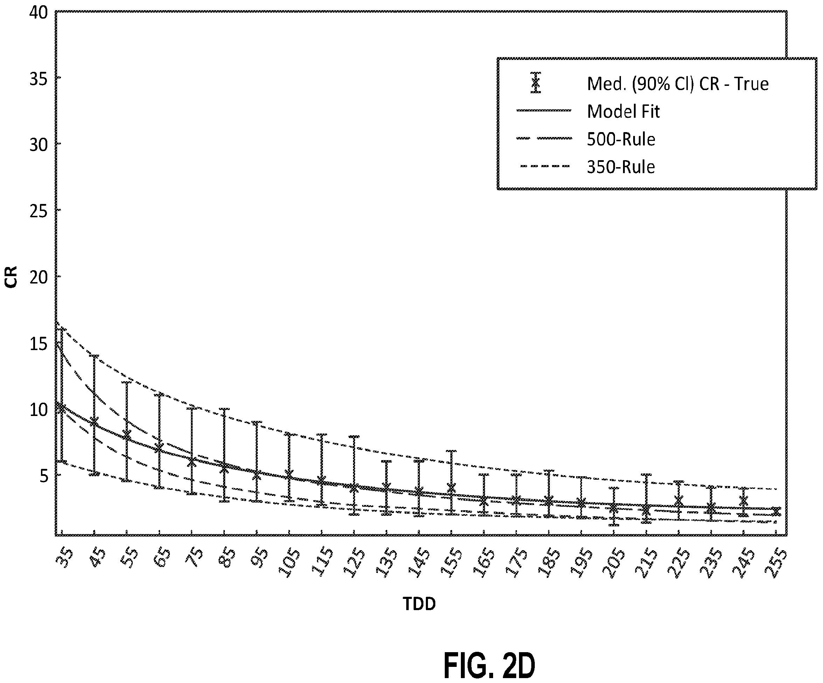

Conventional techniques may utilize what are generally referred to as the 500 Rule, 350 Rule, or the 300 Rule to determine CIR based on TDD for a given patient. For example, using the 500 Rule, a patient's CIR may be determined as 500 divided by the TDD. Similarly, using the 350 Rule, a patient's CIR may be determined as 350 divided by the TDD. This may cause safety and accuracy problems. For example, the CIR determined for relatively low TDDs, such as less than about 30 units per day, may be relatively high. As a more particular example, using the 500 Rule, a CIR of 20 may be calculated for a TDD of 25 units per day, and using the 350 Rule, a CIR of 14 may be calculated for a TDD of 25 units per day. These CIR estimates may be overly conservative (e.g., the CIR may be "too high" than what is required by the patient to accurately calculate insulin dosages), which may lead to elevated blood glucose levels and other undesirable physiological outcomes. Conversely, the 500 Rule and the 350 Rule may lead to CIRs that are relatively low for TDDs that are above some threshold, such as above 35 units per day, above 50 units per day, above 70 units per day, 80 units per day, above 85 units per day, about 90 units per day, above 95 units per day, etc. For example, the 500 Rule and the 350 Rule may lead to CIRs that plateau at about 5 for TDDs within a range of about 85-95 units per day, which may be overly aggressive, thereby causing hypoglycemia. FIG. 2B illustrates comparisons of CIRs determined using the 500 Rule and the 350 Rule to CIRs determined using a population-based approach, as described herein. FIG. 2C illustrates comparisons of CIRs determined using the 500 Rule and the 350 Rule to CIRs determined using a population-based approach for TDDs less than 35 units per day. Note that conventional techniques (e.g., the 500 Rule and the 350 Rule) tend to overestimate CIR for TDDs less than about 35 units per day relative to the population-based techniques described herein. FIG. 2D illustrates comparisons of CIRs determined using the 500 Rule and the 350 Rule to CIRs determined using the population-based approach for TDDs greater than 35 units per day. Note that 350 Rule generates CIRs that are less than those predicted using the population-based techniques described herein for TDDs greater than 35 units per day.

The techniques disclosed herein may lead to more accurate determinations of CIRs for a given patient by utilizing population-level insulin dosages to determine CIRs using, e.g., a model fit to the population-level data. The CIRs determined using the techniques disclosed herein may be evident particularly at relatively low and relatively high TDDs for a given patient compared to the CIRs that may be determined using the conventional 500 Rule the 350 Rule, and/or the 300 Rule. For example, the techniques disclosed herein may lead to a CIR that is less than about 25 grams/unit for a TDD less than about 10 units. As another example, the techniques disclosed herein may lead to a CIR that is greater than about 5 grams/unit for a TDD within a range of about 85 units to 95 units.

In some embodiments, a determination of at least one CIR may be made for a patient based at least in part on a model. Parameters of the model may have been determined based on data associated with a population of patients (e.g., a thousand patients, ten thousand patients, a hundred thousand patients, etc.). In some embodiments, the population of patients may be restricted to those of a similar demographic to the patient for which the CIR is being determined. For example, similar demographic may refer to the population of patients having a similar age (e.g., within ten years, within five years, etc.), or the like. As a more particular example, in an instance in which the patient is a pediatric patient, the model may have been developed using data from a population of pediatric patients. In some implementations, the pediatric patients may additionally be within a particular age range of the patient, e.g., within two years, within four years, within five years, etc. In some embodiments, multiple CIRs may be determined for the patient based on the model. For example, the multiple CIRs may each correspond to a different time of day. It should be noted that, as used herein, two values may be considered "similar" if they are within a predetermined range of each other, where the range may be expressed in percentage (e.g., a first value is less than 5% different from a second value, less than 10% different, etc.), an absolute number (e.g., an absolute value of the difference of two values is less than a predetermined threshold), or any other suitable metric Additionally, for non-numeric criteria, two values may be considered "similar" if they are substantially the same..

In some embodiments, the data associated with the population of patients that is used to develop the model may include, for each patient, at least one CIR used by the patient, and at least one insulin dosage metric. The insulin dosage metric may include a TDD, a total dose from bolus insulin only, a total insulin dosage over a predetermined time period (e.g., a morning insulin dosage, an overnight insulin dosage, etc.), or any combination thereof. Note that, in some embodiments, patients may utilize different CIRs for different time periods, such as a first CIR for morning time periods, a second CIR for afternoon time periods, etc. In such cases, the population data may include multiple CIRs for a given patient represented in the population data.

In some embodiments, the model may comprise one or more mathematical functions that fits data associated with the population of patients. For example, the model may comprise one or more mathematical functions that represent a decreasing CIR at a rate that is in proportion to its current value. As a more particular example, in some embodiments, the one or more mathematical functions may include or may approximate an exponential decay. By way of example, an example mathematical function that may be used to determine a CIR for a patient having a given TDD may be represented as: *CIR = A* * (*e^{-k1*TDD}* + *e^{-k2*TDD}* + *e^{-k3*TDD}*) *+C.* In this equation, *A, k1, k2, k3,* and C represent coefficients that may be fit based on the data associated with the population of patients.

It should be understood that the model may relate any suitable insulin dosage metric to CIR. Additionally, in some embodiments, the model may be a model other than that comprising one or more mathematical functions. For example, the model may be a machine learning model that is trained using a training set comprising the data associated with the population of patients.

FIG. 3 is a flowchart of an example process 300 for determining at least one CIR based at least in part on a model in accordance with some embodiments. Blocks of process 300 may be executed by one or more processors or controller, such as controllers of a delivery device, a, a computing device or system associated with a delivery device, a remote or cloud computing device, or the like. Examples of such devices are shown in and described above in connection with FIG. 1. In some embodiments, blocks of process 300 may be executed in an order other than what is shown in FIG. 3. In some embodiments, two or more blocks of process 300 may be executed substantially in parallel. In some embodiments, one or more blocks of process 300 may be omitted.

Process 300 can begin at 302 by obtaining a metric of insulin dosage associated with a patient. Examples of insulin dosage metrics that may be obtained include a TDD, a total bolus dosage per day, a total insulin delivered over a particular time period or delivered on average over a particular time period (e.g., during particular hours of the day), or the like. Note that, in some embodiments, multiple insulin dosage metrics may be obtained for the patient, such as a TDD and a total bolus dosage per day, a TDD and a total insulin delivered over a particular time period or time of day, a total bolus dosage and a total insulin delivered over a particular time period or time of day, or the like. The insulin dosage metric may be obtained via user input. An example of user input may include patient-input or healthcare-professional provided input, e.g., at a time a new medical device is being initialized (e.g., at a time a new infusion pump is being initialized).

At 304, process 300 can determine at least one CIR for the patient based at least in part on a model. Parameters of the model may have been determined based on data associated with a population of patients. As described above, in some embodiments, the population of patients may include only patients that are similar in at least one demographic characteristic to the patient associated with the obtained metric of insulin dosage. As described above, in some embodiments, the model may comprise one or more mathematical functions that relate at least one insulin dosage metric to a corresponding CIR. For example, as shown in and described above in connection with FIGS. 2A and 2B, the model may comprise one or more mathematical functions that relate a CIR to a TDD. The one or more mathematical functions may include one or more mathematical functions that represent a decreasing carb ratio at a rate that is proportional to its current value. For example, the one or more mathematical functions may include one or more mathematical functions that include and/or approximate an exponential decay. In some embodiments, the parameters of the model may include regression coefficients of the one or more mathematical functions that are determined based at least in part on the data associated with the population of patients.

It should be noted that, in some embodiments, the model may include multiple sets of mathematical functions, each associated with a different percentile of CIRs for the population of patients. For example, the model may include a first set of mathematical functions that represents the median CIR for the population of patients as a function of the insulin dosage metric (e.g., of the TDD, as shown in and described above in connection with FIGS. 2A and 2B). As another example, the model may include second and third sets of mathematical functions that represent other percentiles of CIR for the population of patients as a function of the insulin dosage metric (e.g., 10^{th} and 90^{th} percentiles, as shown in and described above in connection with FIGS. 2A and 2B). In some implementations, model fits for any suitable number of percentiles may be determined (e.g., for three percentiles such as the 10^{th}, 50^{th}, and 90^{th} percentiles, nine percentiles such as the 10^{th}-90^{th} percentiles in increments of ten units, or the like).

In some embodiments, outputs of the model (e.g., outputs of the one or more mathematical functions) may be stored in a table, e.g., a look-up table. The look-up table may use the insulin dosage metric as a key, with corresponding values for the CIR represented as multiple potential values. For example, given a TDD of, e.g., 35 units/day, the look-up table may indicate that the 10^{th} percentile CIR is 7.5 grams/unit, the median CIR is 10 grams/unit, and the 90^{th} percentile CIR is 15 grams/unit. In some embodiments, the at least one CIR may be identified using such a look-up table.

In some embodiments, after determining the at least one CIR, process 300 may present an indication of the at least one CIR, e.g., to a patient or a healthcare provider for confirmation that the at least one CIR is to be used to determine insulin dosing. In some embodiments, process 300 may cause the at least one CIR to be used as a medical device parameter going forward for determination of insulin dosing. For example, the at least one CIR may be used to initialize settings and/or modify settings for the medical device.

Note that, in some embodiments, the at least one CIR that is obtained may be determined based at least in part on an operating mode of the medical device (e.g., of the infusion pump). For example, in some embodiments, responsive to a determination that the operating mode is a fully-closed loop operating mode (e.g., where the patient need not enter meal-related information such as number of carbohydrates in a meal or that a meal is being consumed), the at least one CIR may be associated with relatively lower percentiles of the model fit (e.g., a 10^{th} percentile, a 20^{th} percentile, less than the 50^{th} percentile, or the like). Selection of a CIR associated with a relatively lower percentile may allow for more aggressive insulin dosing, which may be safer with a fully-closed operating mode than with a device operating under a manual or hybrid operating mode.

At 330, process 300 may optionally provide an indication of a range of CIRs associated with the population for the insulin dosage metric associated with the patient. For example, in some embodiments, process 300 may indicate CIRs associated with particular upper and lower percentiles, such as the 10^{th} and 90^{th} percentiles, the 25^{th} and 75^{th} percentiles, etc. In such cases, providing a range of CIRs within a particular percentile bound may allow a patient and/or a healthcare provider insight into how conservative or aggressive a particular CIR is

Note that the indication of the at least one CIR, and/or the range of CIRs associated with the population, may be provided via a user interface, e.g., a user interface associated with the medical device, a monitoring device, a paired mobile device, or the like.

Optionally, at 340, process 300 can cause insulin to be delivered to the patient based at least in part on the CIR determined at 320. For example, in some embodiments, process 300 can determine an insulin dosage (e.g., a bolus insulin dosage) based on the CIR, e.g., responsive to an indication of a number of carbohydrates in a consumed meal or snack. In some embodiments, causing insulin to be delivered to the patient may comprise transmitting instructions that actuate an infusion pump to deliver the dosage of insulin determined based on the CIR.

As described above in connection with FIG. 2B, CIRs determined using the techniques disclosed herein may differ from CIRs determined using conventional techniques, such as those determined using the 500 Rule the 350 Rule, and/or the 300 Rule. In particular, the CIRs may vary at relatively low TDDs (e.g., less than about 35 units), and at relatively high TDDs (e.g., greater than about 85 grams/unit, greater than about 95 grams/unit, etc.). As a more particular example, using the techniques disclosed herein, a CIR may be determined for a TDD of 10 units or less that is less than 25 grams/unit, whereas a CIR for a TDD of 10 units may conventionally be determined to be greater than 25 grams/unit (e.g., as determined using the 500 Rule the 350 Rule, or the 300 Rule). As another more particular example, using the techniques disclosed herein, a CIR may be determined for a TDD of 25 units or less that is greater than about 12 grams/unit, whereas a CIR for a TDD of 25 units may conventionally be determined to be greater than 12 grams/unit. As another more particular example, using the techniques disclosed herein, a CIR for a TDD within a range of about 85 - 95 units may be determined to be greater than 5 grams/unit, whereas CIR may plateau at 5 grams/unit when using the 500 Rule the 350 Rule, or the 300 Rule.

FIG. 4 is a flowchart of an example process 400 for determining at least one CIR for a patient using an obtained TDD in accordance with some embodiments. Blocks of process 400 may be executed by one or more processors or controller, such as controllers of a delivery device, a monitoring device, a computing device or system, or the like. Examples of such devices are shown in and described above in connection with FIG. 1. In some embodiments, blocks of process 400 may be executed in an order other than what is shown in FIG. 4. In some embodiments, two or more blocks of process 400 may be executed substantially in parallel. In some embodiments, one or more blocks of process 400 may be omitted.

Process 400 can begin at 410 by obtaining a TDD of insulin for a patient. The TDD may be obtained by user input, e.g., by the patient, by a healthcare provider, etc. The TDD may be obtained at initialization or set-up of the medical device, e.g., initialization of an infusion pump.

At 420, process 400 can determine at least one CIR for the patient, where the CIR is less than about 25 grams/unit for a TDD less than 10 units, or where the CIR is greater than about 5 grams/unit for a TDD less than about 105 units. In other words, in instances in which the TDD obtained at block 410 is less than about 10 units, the CIR determined at block 420 is less than about 25 grams/unit, and, in instances in which the TDD obtained at block 410 is less than 105 units, the CIR determined at block 420 is greater than about 5 grams/unit. In some embodiments, the CIR may differ from a CIR that would be predicted by the 500 Rule the 350 Rule, or the 300 Rule for other TDDs. For example, in instances in which the TDD obtained at block 410 is less than about 25 units, the CIR may be less than about 13 grams/unit. As another example, in instances in which the TDD obtained at block 410 is less than about 15 units, the CIR may be less than about 20 grams/unit.

Optionally, at 430, process 400 can cause insulin to be delivered to the patient based at least in part on the CIR determined at 420. For example, in some embodiments, process 400 can determine an insulin dosage (e.g., a bolus insulin dosage) based on the CIR, e.g., responsive to an indication of a number of carbohydrates in a consumed meal or snack. In some embodiments, causing insulin to be delivered to the patient may comprise transmitting instructions that actuate an infusion pump to deliver the dosage of insulin determined based on the CIR.

### Example Apparatus

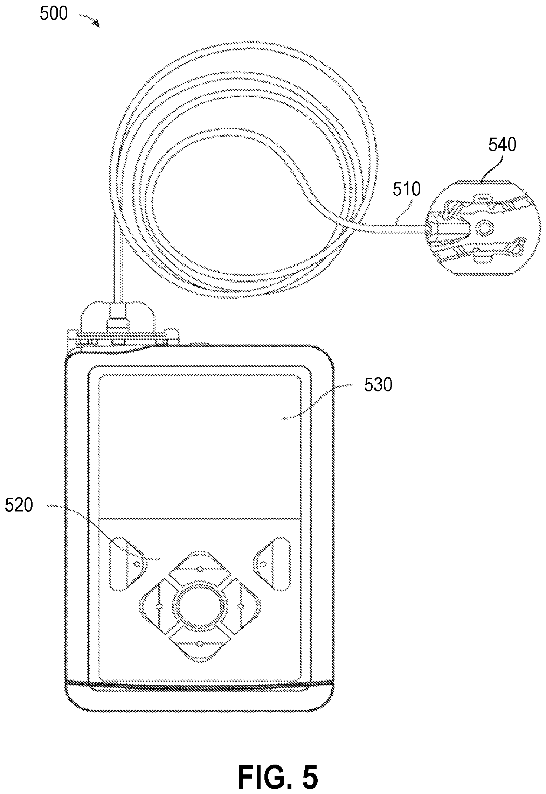

In some embodiments, therapy may be effected based on communicating a therapy determination toward a therapy delivery device. A non-limiting example of such a device is described below in connection with FIG. 5, which depicts an example insulin delivery device 500, in accordance with aspects of the present disclosure.

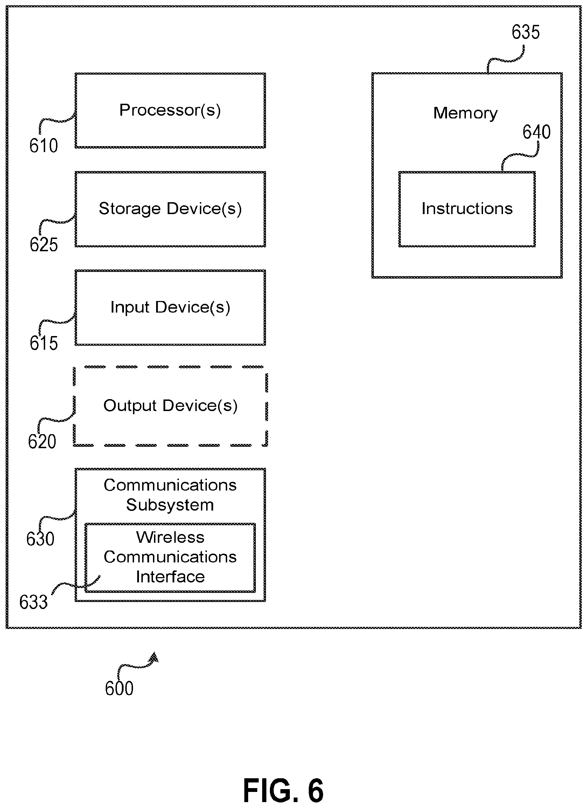

As mentioned above, therapy determinations may be communicated toward an insulin delivery device 500 (e.g., from a cloud computing system 108 via an intermediary computing device 106 communicatively coupled to the device 500). The insulin delivery device 500 may be an example of the delivery device 102 as described throughout this disclosure. In such a device, insulin delivery may be performed based on internal communication between a central computing module (e.g., a microcontroller for device 500 as a whole) and an insulin delivery module (e.g., including a motor and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., 106, FIG. 1) communicatively coupled to a remote or cloud computing system (e.g., 108, FIG. 1). The insulin delivery device 500 may communicate various event data (e.g., meal data, exercise data, and/or insulin delivery data) toward the remote or cloud computing system, which may communicate insulin delivery determinations toward the insulin delivery device 500, in some implementations. FIG. 6 further illustrates components which can be included in the insulin delivery device 500.

The insulin delivery device 500 can provide fast-acting insulin through a small tube 510 configured for fluidic connection with a cannula (not shown). The cannula may be inserted subcutaneously under a fixation dressing 540 that includes an inlet for the tube 510, an outlet for the cannula, and an adhesive surface for affixing the dressing 540 to skin. The device 500 can deliver at least two types of dosages-a basal dosage, which can be delivered periodically (e.g., every five minutes) in tiny amounts throughout the day and night, and a bolus dosage to cover an increase in blood glucose from meals and/or to otherwise correct high blood glucose levels. The depicted insulin delivery device 500 includes a user interface having button elements 520 that can be manipulated to administer a bolus of insulin, to change therapy settings, to change user preferences, to select display features, and the like. The insulin delivery device 500 also includes a display device 530 that can be used to present various types of information or data to the user (such as a notification or an alert of the type described above). In accordance with aspects of the present disclosure, a user of the insulin delivery device 500 may use the button elements 520 to input certain event data (e.g., event type, event start time, event details, etc.), and the user inputs can be confirmed using the display device 530. The depicted insulin delivery device 500 of FIG. 5 is merely provided by way of example, and other types of insulin delivery devices and other techniques different from those described above are contemplated to be within the scope of the present disclosure.

FIG. 6 is a block diagram of an embodiment of a computer system 600, which can be utilized in embodiments as described herein. It should be noted that FIG. 6 is meant only to provide a generalized illustration of various components, any or all of which may be utilized as appropriate. In addition, it can be noted that components illustrated by FIG. 6 can be localized to a single device (e.g., delivery device 102, monitoring device 104, computing device 106, or computing system 108) and/or distributed among various networked devices, which may be disposed at different geographical locations.

In some embodiments, the computer system 600 may include hardware elements that can be electrically coupled via a bus (or may otherwise be in communication, as appropriate). The hardware elements may include processor(s) 610, which may comprise, without limitation, one or more microcontroller(s), one or more microprocessor(s), one or more general-purpose processors, one or more special-purpose processors (such as digital signal processing chips, graphics acceleration processors, and/or the like), and/or other processing structure, which can be configured to perform one or more of the methods or functionalities described herein.

In some embodiments, the computer system 600 also may include one or more input devices 615, which may comprise, without limitation, button elements 520, a microphone, a glucose sensor, and/or the like. The computer system 600 may also include one or more output devices 620, which may comprise without limitation a display device (e.g., 530), a speaker, a buzzer, and/or the like.

In some embodiments, the computer system 600 may further include one or more non-transitory storage devices 625, which can include, without limitation, local and/or network accessible storage, and/or may comprise, without limitation, a disk drive, a drive array, an optical storage device, a solid-state storage device, such as a read-access memory (RAM) and/or read-only memory (ROM), which can be programmable, flash-updateable, and/or the like. Such storage devices may be configured to implement any appropriate data stores, including without limitation, various file systems, database structures, and/or the like. Such data stores may include database(s) and/or other data structures used to store and administer messages and/or other information to be sent to one or more other components or external devices.

In some embodiments, the computer system 600 may also include a communications subsystem 630, which may implement wireless communication technologies managed and controlled by a wireless communication interface 633. Additionally or alternatively, communications subsystem 630 may implement wired technologies (such as Ethernet, coaxial communications, universal serial bus (USB), or the like). The wireless communication interface 633 may comprise one or more wireless transceivers that may send and receive wireless signals (e.g., signals according to Bluetooth, Bluetooth Low Energy (BLE)). Thus, the communications subsystem 630 may comprise a modem, a network card (wireless or wired), an infrared communication device, a wireless communication device, and/or a chipset, and/or the like, which may enable the computer system 600 to communicate with any device discussed with respect to FIG. 1, including delivery device 102, monitoring device 104, computing device 106, and/or a cloud computing system 108 as described herein. Hence, the communications subsystem 630 may be used to receive and send data (e.g., SG, Ip, insulin delivery information) as described in the embodiments herein.

In some embodiments, the computer system 600 will further comprise a working memory 635, which may comprise a RAM or ROM device, as described above. Software elements, shown as being located within the working memory 635, may comprise computer-readable and computer-executable instructions 640; device drivers; executable libraries; and/or other code, which may comprise computer programs used in various embodiments and/or may be designed to implement methods and/or configure systems in accordance with embodiments described herein. Merely by way of example, one or more operations described with respect to the methods or functionalities discussed above might be implemented as code and/or instructions executable by a computer (and/or a processor within a computer). Such code and/or instructions can be used to configure and/or adapt a general-purpose computer (or other device) to perform one or more operations in accordance with the described methods.

In some embodiments, a set of these instructions and/or code may be stored on a non-transitory computer-readable storage medium, such as the storage device(s) 625 described above. In some cases, the storage medium might be incorporated within a computer system, such as computer system 600. In other embodiments, the storage medium might be separate from a computer system (e.g., a removable medium, such as an optical disc) and/or provided in a downloadable installation package, such that the storage medium can be used to program, configure, and/or adapt a general purpose computer with the instructions and/or code stored thereon. These instructions might take the form of executable code, which is executable by the computer system 600, and/or might take the form of source and/or installable code, which, upon compilation and/or installation on the computer system 600 (e.g., using any of a variety of generally available compilers, installation programs, compression/decompression utilities, etc.), then takes the form of executable code.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

Example Embodiments:
Embodiment 1: A method of determining medical device parameters, the method comprising: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.
Embodiment 2: The method of embodiment 1, wherein the metric of insulin dosage is at least one of: a total daily dose (TDD) of insulin, a total bolus dosage of insulin, or a total dose within a predetermined time period.
Embodiment 3: The method of any of the foregoing embodiments, wherein the data associated with the population of patients comprises, for a given patient in the population, an insulin dosage metric associated with the given patient and at least one carbohydrate-to-insulin ratio used by the patient.
Embodiment 4: The method of any of the foregoing embodiments, wherein the population of patients comprise a set of patients of a similar demographic group as the patient.
Embodiment 5: The method of embodiment 4, wherein the similar demographic group comprises the set of patients being of a similar age as the patient.
Embodiment 6: The method of any of the foregoing embodiments, wherein the model comprises one or more mathematical functions that represent a decreasing carbohydrate-to-insulin ratio at a rate that is proportional to its current value.
Embodiment 7: The method of embodiment 6, wherein the one or more mathematical functions approximates an exponential decay.
Embodiment 8: The method of any of the foregoing embodiments, further comprising providing an indication of a range of carbohydrate-to-insulin ratios spanning a predetermined confidence interval based on the data associated with the population of patients.
Embodiment 9: The method of embodiment 8, wherein the indication of the range of carbohydrate-to-insulin ratios is provided when initializing or modifying use of an insulin infusion pump.
Embodiment 10: The method of any of the foregoing embodiments, wherein the obtaining the metric of insulin dosage for the patient is during operation of an insulin infusion pump, and wherein the determined at least one carbohydrate-to-insulin ratio is used to modify programming of the insulin infusion pump.
Embodiment 11: The method of any of the foregoing embodiments, wherein the at least one carbohydrate-to-insulin ratio for the patient is determined based at least in part on an operating mode of an insulin infusion pump to use the at least one carbohydrate-to-insulin ratio, and wherein the operating mode of the insulin infusion pump comprises at least one of: a manual mode, or a fully-closed loop mode.
Embodiment 12: The method of any of the foregoing embodiments, wherein the at least one carbohydrate-to-insulin ratio for the patient comprises a plurality of carbohydrate-to-insulin ratios, each carbohydrate-to-insulin ratio applicable to a predetermined time of day.
Embodiment 13: A system comprising: one or more processors; and one or more processor-readable media storing instructions which, when executed by the one or more processors, cause performance of: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.
Embodiment 14: The system of embodiment 13, wherein the model comprises one or more mathematical functions that represent a decreasing carbohydrate-to-insulin ratio at a rate that is proportional to its current value.
Embodiment 15: One or more processor-readable media storing instructions which, when executed by one or more processors, cause performance of: obtaining a metric of insulin dosage for a patient; and determining at least one carbohydrate-to-insulin ratio for the patient based at least in part on a model and using the obtained metric of insulin dosage for the patient, wherein parameters of the model were determined based on data associated with a population of patients.
Embodiment 16: A method of determining medical device parameters, the method comprising: obtaining a total daily dose (TDD) of insulin for a patient; and determining at least one carbohydrate-to-insulin ratio for the patient, wherein the carbohydrate-to-insulin ratio is less than 25 grams/unit for a TDD less than 10 units, and wherein the carbohydrate to insulin ratio is greater than 5 grams/unit for a TDD for a TDD less than 105 units.
Embodiment 17: The method of embodiment 16, wherein the at least one carbohydrate-to-insulin ratio is obtained from a look-up table.
Embodiment 18: The method of embodiment 17, wherein the look-up table includes a plurality of potential carbohydrate-to-insulin ratios for a given TDD, each potential carbohydrate-to-insulin ratio applicable to a different percentile of a set of patients used to generate the look-up table.
Embodiment 19: The method of embodiment 18, further comprising providing an alert or an indication of carbohydrate-to-insulin ratios applicable to different percentiles.
Embodiment 20: The method of any one of embodiments 18 or 19, wherein the set of patients comprises patients of a similar demographic group as the patient.
Embodiment 21: The method of any of embodiments 16-20, further comprising causing insulin to be delivered to the patient based at least in part on the determined at least one carbohydrate-to-insulin ratio.

Further disclosed herein is the subject-matter of the following clauses:
1. A method of determining medical device parameters, the method comprising:
   obtaining a metric of insulin dosage for a patient; and
   determining at least one carbohydrate-to-insulin ratio for the patient based at least in part on a model and using the obtained metric of insulin dosage for the patient, wherein parameters of the model were determined based on data associated with a population of patients.
2. The method of clause 1, wherein the metric of insulin dosage is at least one of: a total daily dose (TDD) of insulin, a total bolus dosage of insulin, or a total dose within a predetermined time period.
3. The method of clause 1 or 2, wherein the data associated with the population of patients comprises, for a given patient in the population, an insulin dosage metric associated with the given patient and at least one carbohydrate-to-insulin ratio used by the patient.
4. The method of clause 1 or of one of clauses 1 to 3, wherein the population of patients comprise a set of patients of a similar demographic group as the patient.
5. The method of clause 4 or of one of clauses 1 to 4, wherein the similar demographic group comprises the set of patients being of a similar age as the patient.
6. The method of clause 1 or of one of clauses 1 to 5, wherein the model comprises one or more mathematical functions that represent a decreasing carbohydrate-to-insulin ratio at a rate that is proportional to its current value.
7. The method of clause 6 or of one of clauses 1 to 6, wherein the one or more mathematical functions approximates an exponential decay.
8. The method of clause 1 or of one of clauses 1 to 7, further comprising providing an indication of a range of carbohydrate-to-insulin ratios spanning a predetermined confidence interval based on the data associated with the population of patients.
9. The method of clause 8 or of one of clauses 1 to 8, wherein the indication of the range of carbohydrate-to-insulin ratios is provided when initializing or modifying use of an insulin infusion pump.
10. The method of clause 1 or of one of clauses 1 to 9, wherein the obtaining the metric of insulin dosage for the patient is during operation of an insulin infusion pump, and wherein the determined at least one carbohydrate-to-insulin ratio is used to modify programming of the insulin infusion pump.
11. The method of clause 1 or of one of clauses 1 to 10, wherein the at least one carbohydrate-to-insulin ratio for the patient is determined based at least in part on an operating mode of an insulin infusion pump to use the at least one carbohydrate-to-insulin ratio, and wherein the operating mode of the insulin infusion pump comprises at least one of: a manual mode, or a fully-closed loop mode.
12. The method of clause 1 or of one of clauses 1 to 11, wherein the at least one carbohydrate-to-insulin ratio for the patient comprises a plurality of carbohydrate-to-insulin ratios, each carbohydrate-to-insulin ratio applicable to a predetermined time of day.
13. A system comprising:
   one or more processors; and
   one or more processor-readable media storing instructions which, when executed by the one or more processors, cause performance of:
      obtaining a metric of insulin dosage for a patient; and
      determining at least one carbohydrate-to-insulin ratio for the patient based at least in part on a model and using the obtained metric of insulin dosage for the patient, wherein parameters of the model were determined based on data associated with a population of patients.
14. The system of clause 13, wherein the model comprises one or more mathematical functions that represent a decreasing carbohydrate-to-insulin ratio at a rate that is proportional to its current value.
15. A method of determining medical device parameters, the method comprising:
   obtaining a total daily dose (TDD) of insulin for a patient; and
   determining at least one carbohydrate-to-insulin ratio for the patient, wherein the carbohydrate-to-insulin ratio is less than 25 grams/unit for a TDD less than 10 units, and wherein the carbohydrate to insulin ratio is greater than 5 grams/unit for a TDD for a TDD less than 105 units.
16. The method of clause 15, wherein the at least one carbohydrate-to-insulin ratio is obtained from a look-up table.
17. The method of clause 15 or 16, wherein the look-up table includes a plurality of potential carbohydrate-to-insulin ratios for a given TDD, each potential carbohydrate-to-insulin ratio applicable to a different percentile of a set of patients used to generate the look-up table.
18. The method of clause 17 or of one of clauses 15 to 17, wherein the set of patients comprises patients of a similar demographic group as the patient.
19. The method of clause 17 or of one of clauses 15 to 18, further comprising providing an alert or an indication of carbohydrate-to-insulin ratios applicable to different percentiles.
20. The method of clause 15 or of one of clauses 15 to 19, further comprising causing insulin to be delivered to the patient based at least in part on the determined at least one carbohydrate-to-insulin ratio.

## Claims

1. A method of determining medical device parameters, the method comprising:
obtaining a metric of insulin dosage for a patient; and
determining at least one carbohydrate-to-insulin ratio for the patient based at least in part on a model and using the obtained metric of insulin dosage for the patient, wherein parameters of the model were determined based on data associated with a population of patients.

2. The method of claim 1, wherein the metric of insulin dosage is at least one of: a total daily dose (TDD) of insulin, a total bolus dosage of insulin, or a total dose within a predetermined time period.

3. The method of claim 1 or 2, wherein the data associated with the population of patients comprises, for a given patient in the population, an insulin dosage metric associated with the given patient and at least one carbohydrate-to-insulin ratio used by the patient.

4. The method of one of claims 1 to 3, wherein the population of patients comprise a set of patients of a similar demographic group as the patient, particularly
wherein the similar demographic group comprises the set of patients being of a similar age as the patient.

5. The method of one of claims 1 to 4, wherein the model comprises one or more mathematical functions that represent a decreasing carbohydrate-to-insulin ratio at a rate that is proportional to its current value, particularly
wherein the one or more mathematical functions approximates an exponential decay.

6. The method of one of claims 1 to 5, further comprising providing an indication of a range of carbohydrate-to-insulin ratios spanning a predetermined confidence interval based on the data associated with the population of patients, particularly
wherein the indication of the range of carbohydrate-to-insulin ratios is provided when initializing or modifying use of an insulin infusion pump.

7. The method of one of claims 1 to 6, wherein the obtaining the metric of insulin dosage for the patient is during operation of an insulin infusion pump, and wherein the determined at least one carbohydrate-to-insulin ratio is used to modify programming of the insulin infusion pump.

8. The method of one of claims 1 to 7, wherein the at least one carbohydrate-to-insulin ratio for the patient is determined based at least in part on an operating mode of an insulin infusion pump to use the at least one carbohydrate-to-insulin ratio, and wherein the operating mode of the insulin infusion pump comprises at least one of: a manual mode, or a fully-closed loop mode.

9. The method of one of claims 1 to 8, wherein the at least one carbohydrate-to-insulin ratio for the patient comprises a plurality of carbohydrate-to-insulin ratios, each carbohydrate-to-insulin ratio applicable to a predetermined time of day.

10. A system comprising:
one or more processors; and
one or more processor-readable media storing instructions which, when executed by the one or more processors, cause performance of:
obtaining a metric of insulin dosage for a patient; and
determining at least one carbohydrate-to-insulin ratio for the patient based at least in part on a model and using the obtained metric of insulin dosage for the patient, wherein parameters of the model were determined based on data
associated with a population of patients, particularly
wherein the model comprises one or more mathematical functions that represent a decreasing carbohydrate-to-insulin ratio at a rate that is proportional to its current value.

11. A method of determining medical device parameters, the method comprising:
obtaining a total daily dose (TDD) of insulin for a patient; and
determining at least one carbohydrate-to-insulin ratio for the patient, wherein the carbohydrate-to-insulin ratio is less than 25 grams/unit for a TDD less than 10 units, and wherein the carbohydrate to insulin ratio is greater than 5 grams/unit for a TDD for a TDD less than 105 units.

12. The method of claim 11, wherein the at least one carbohydrate-to-insulin ratio is obtained from a look-up table.

13. The method of claim 12, wherein the look-up table includes a plurality of potential carbohydrate-to-insulin ratios for a given TDD, each potential carbohydrate-to-insulin ratio applicable to a different percentile of a set of patients used to generate the look-up table.

14. The method of claim 13, wherein the set of patients comprises patients of a similar demographic group as the patient, and/or
further comprising providing an alert or an indication of carbohydrate-to-insulin ratios applicable to different percentiles.

15. The method of one of claim 11 to 14, further comprising causing insulin to be delivered to the patient based at least in part on the determined at least one carbohydrate-to-insulin ratio.
